# EUROPEAN PATENT APPLICATION

(11) **EP 1 992 632 A1**
(43) Date of publication of application: **19.11.2008**
(21) Application number: 07009579.9
(22) Date of filing: 12.05.2007
(51) Int. Cl.: C07H 19/09

(54) **Method for the synthesis of cyclouridine**

(71) Applicant: EXPLORA Laboratories SA, 6850 Mendrisio (CH)
(72) Inventor: Mociardini, Simone, 21100 Varese (IT); Colombo, Paolo, 21013 Gallarate (VA) (IT)
(74) Representative: Zardi, Marco

(57) **Abstract**

Method for the synthesis of possibly substituted cyclic derivatives of uridine, of formula (II) 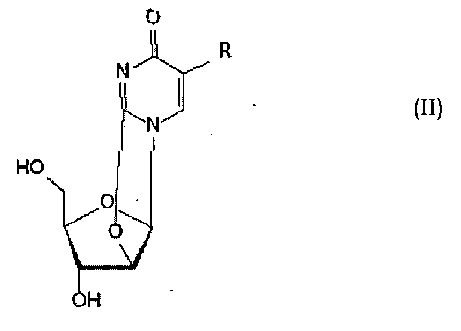 wherein R is chosen in the group that comprises: hydrogen, halogen and C₁₋₄ alkyl, the method comprising the step of reacting a possibly substituted uridine of formula (III) 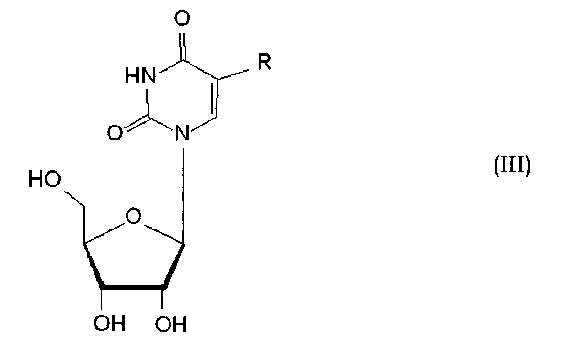 wherein R has the same meanings as in formula (II), and glycerol carbonate in the presence of an alkaline carbonate at a temperature of 70°C-130°C.

## Description

### Field of application

The present invention refers to a method for the synthesis of cyclic derivatives of uridine.

More specifically, the invention refers to a method for the synthesis of cyclouridine and its substituted derivatives.

### Prior art

As it is well known, O²,2'-Cyclouridine, (formula (I)), known as cyclouridine) is a molecule of molecular formula C₉H₁₀N₂O₅ and having a molecular weight of 226.187.

Cyclouridine is commonly used as a key reaction intermediate for the synthesis of uridine derivatives such as arabinofuranosyl-uracil (ARA-U), 2'-deoxyuridine and 2',3'-dideoxyuridine, which are in turn key starting materials for the production of many antiviral or antitumour active ingredients of nucleoside origin.

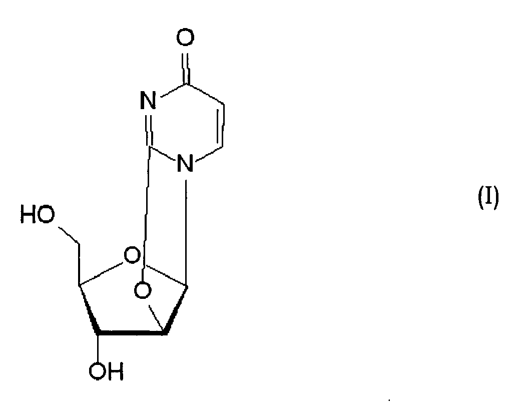

Various methods for the synthesis of cyclouridine are already known and described in the field.

The method most commonly used involves the use of a cyclic carbonate (for example dimethylcarbonate, diethylcarbonate, diphenylcarbonate, ethylenecarbonate) in the presence of dimethylformamide (DMF) or other high-boiling point solvents and of sodium bicarbonate. This process, at the end of the reaction, then generally involves a work-up with distillation of the residual solvent and subsequent crystallisation by taking up the residue with alcohols.

However, such a process involves some drawbacks.

A drawback related to the use of such carbonates lies in the fact that they require the use of reaction solvents (generally dimethylformamide (DMF) or other high-boiling point solvents) that are very toxic.

Moreover, another problem linked to the use of the carbonates normally used, for example diphenylcarbonate, concerns their decomposition products, which are often toxic. Moreover, such toxic decomposition products can be difficult to remove from the end product in the distillation step.

In other cases, the decomposition product can act, as in the case of dimethylcarbonate and diethylcarbonate, to the detriment of the yields of reaction product, as it lowers the reaction temperature. This, in turn, results in the need to use a greater amount of solvent (for example DMF) in order for the reaction to proceed to completion. Consequently, the reaction uses a substantially greater amount of toxic solvents and the reaction time (10-12 hours in the case of diethylcarbonate) is also increased.

In some cases, such carbonates are irritant agents, like in the case of ethylenecarbonate, and are therefore difficult to handle. This leads to an intrinsic difficulty in their processing.

The solvents used must then be removed during the distillation step, thus making the processing more complicated.

The technical problem at the basis of the present invention is therefore that of providing a method for the synthesis of cyclouridine and/or its cyclic derivatives at yields comparable to or greater than those of the prior art, having a low environmental impact and which starts from raw materials that are not toxic and that are easily available, and that is at the same time economically advantageous, technically rapid and easy to carry out.

### Summary of the invention

Such a problem is solved according to the present invention by a method for the synthesis of possibly substituted cyclic derivatives of uridine, of formula (II) 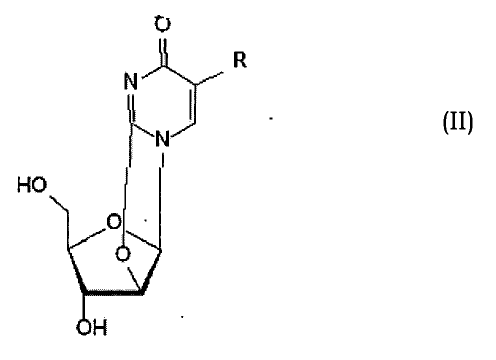 wherein R is chosen in the group that comprises: hydrogen, halogen, and C₁₋₄ alkyl, such a method comprising the step of reacting a possibly substituted uridine of formula (III) 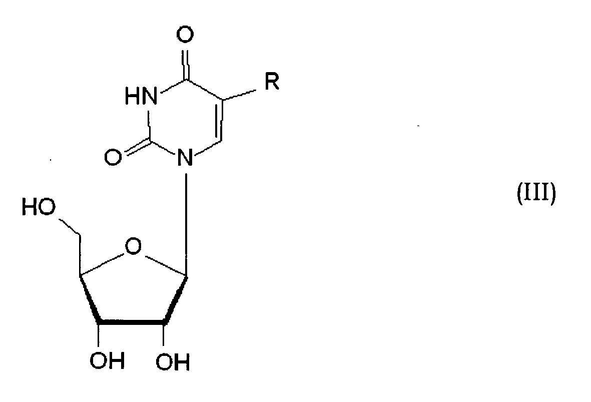 wherein R has the same meanings as in formula (II), and glycerol carbonate in the presence of an alkaline carbonate at a temperature of 70-130°C.

Preferably, R is chosen in the group that comprises: hydrogen, fluorine, ethyl and methyl.

Preferably, the possibly substituted uridine of formula (III) and the glycerol carbonate are made to react in a w/v ratio that is variable between 1:10 and 1:2, preferably between 1:5 and 2:3, advantageously in a w/v ratio of 1:3.

The reaction time is generally between 0.5 and 4 hours.

Preferably, the reaction between possibly substituted uridine and glycerol carbonate is carried out at a temperature comprised between 90 and 110°C for 1-3 hours.

According to a more preferred embodiment of the present invention, the reaction between possibly substituted uridine and glycerol carbonate is carried out at a temperature of 100°C for 2 hours.

Preferably the aforementioned alkaline carbonate is sodium carbonate.

The reaction product can be separated from the final reaction mixture by means of conventional techniques.

Preferably, the separation of the cyclic derivative of the possibly substituted uridine from the mixture comprises the steps of: concentration, preferably under vacuum of the mixture at a temperature comprised between 50 and 70°C, preferably at 60°C; cooling of the mixture; and dilution in an alcohol, preferably methanol.

Preferably, following such a separation, the cyclic derivative of the uridine thus produced is filtered and dried according to conventional methods.

The present invention also comprises a method for the synthesis of derivatives of arabinofuranosyl-uracil of formula (IV) 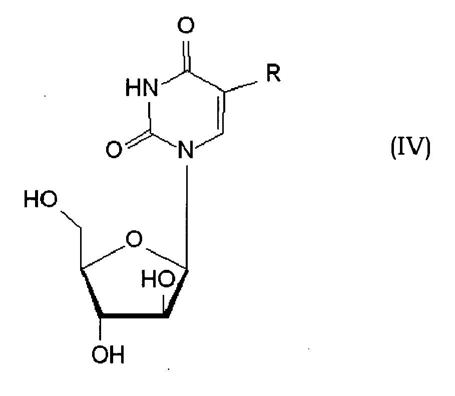 wherein R is chosen in the group that comprises: hydrogen, halogen and C₁₋₄ alkyl, the method comprising the steps of:
a) Converting a possibly substituted uridine of formula (III) 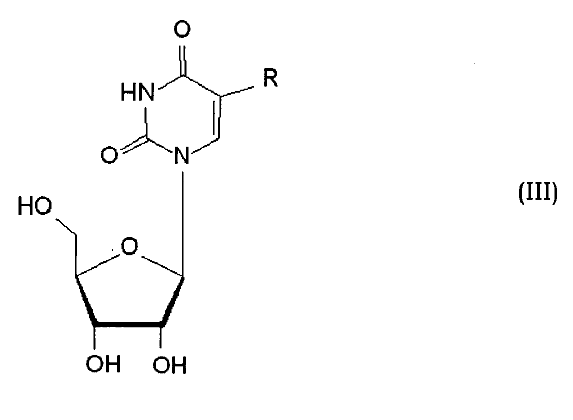 wherein R has the same meanings as above, to a possibly substituted cyclic derivative of uridine of formula (II); 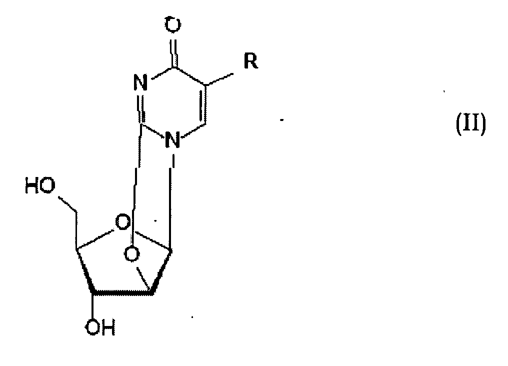
b) Reacting said possibly substituted cyclic derivative of uridine of formula (II) with an alkaline hydroxide in aqueous solution;
characterised in that said reaction of step a) is carried out by reacting said possibly substituted uridine of formula (III) wherein R has the same meanings as in formula (II), and glycerol carbonate in the presence of an alkaline carbonate at a temperature of 70-130°C.

Preferably, R is chosen in the group that comprises: hydrogen, fluorine, ethyl and methyl.

Preferably, the possibly substituted uridine of formula (III) and the glycerol carbonate are made to react in a w/v ratio that is variable between 1:10 and 1:2, preferably between 1:5 and 2:3, advantageously in a w/v ratio of 1:3.

The reaction time is generally comprised between 0.5 and 4 hours.

Preferably, the reaction between possibly substituted uridine and glycerol carbonate is carried out at a temperature comprised between 90 and 110°C for 1-3 hours.

According to a more preferred embodiment of the present invention, the reaction between possibly substituted uridine and glycerol carbonate is carried out at a temperature of 100°C for 2 hours.

Preferably, sodium carbonate is used as the alkaline carbonate.

Preferably, the reaction of step b) is carried out at room temperature for 15-25 hours, preferably for 18-20 hours.

Preferably, NaOH is used as the alkaline hydroxide.

Step b) is followed by a final separation step of the arabinofuranosyl-uracil derivatives from the reaction mixture.

Preferably, the separation of the aforementioned derivatives from the reaction mixture comprises the steps of: filtering the mixture; concentration, preferably under vacuum of the mixture; and cooling of the mixture at 4°C overnight.

Preferably, such a concentration is carried out until the volume is reduced to a quarter of the initial volume.

The method according to the present invention involves the use of glycerol carbonate, until now never used in the synthesis of cyclic derivatives of uridine, as reagent and reaction solvent.

The use of glycerol carbonate in the synthesis of possibly substituted cyclic derivatives of uridine, in place of the carbonates usually used in the synthesis of cyclouridine (such as, for example, dimethylcarbonate, diethylcarbonate, diphenylcarbonate, ethylenecarbonate), offers the advantage that it acts both as a reactant and as a reaction solvent.

This way, it is no longer necessary to use an additional solvent, such as those usually used in the known methods, such as, for example, DMF or other high-boiling point solvents, which are usually toxic.

The method of synthesis of cyclic derivatives, possibly substituted, of the uridine through the use of glycerol carbonate therefore has a lesser environmental impact compared to the known methods.

Moreover, glycerol carbonate itself has a low ecological impact since its decomposition product is simply glycerine.

Moreover, glycerol carbonate is obtained from the processing of the waste products of the production of biodiesel fuels and is therefore easily available and inexpensive.

Unlike known methods, in the separation step of the reaction product from the mixture according to the present invention, there is no need to remove large amounts of solvents, since it is sufficient to remove the unreacted glycerol carbonate residue. Glycerine, in fact, unlike DMF, does not interfere with precipitation.

### Detailed description

The present invention shall now be described further with reference to some example embodiments provided hereafter for illustrative and not limiting purposes.

### EXAMPLE No. 1. Synthesis of cyclouridine

220 grams of Uridine (MW 244.202) were reacted in 660 ml of glycerolcarbonate, in the presence of 3.3 g of Sodium Carbonate, for 2 hours at 100°C, and the product synthesis was monitored using HPLC (see the diagram below)

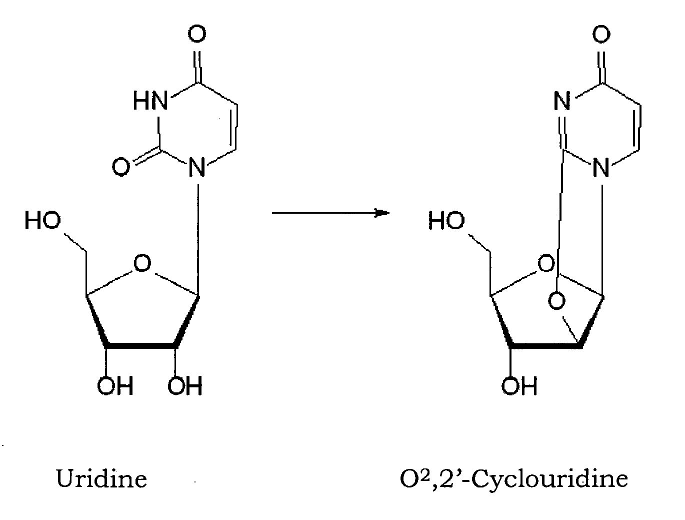

At the end of the reaction the reaction mixture (containing crystals in suspension) formed was concentrated under vacuum at 60°C to remove the excess solvent. The residue was then taken up with methanol resulting in further crystal formation.

After cooling at 4°C overnight, the crystalline solid was separated by filtration and dried thus obtaining 152g of Cyclouridine (MW 226.187) with a purity measured by HPLC of 99.9%. The yield (w/w) was thus of 74.6%.

The reaction has a duration of two hours and can be carried out at a lower temperature than the one used in the known methods.

Its processing according to the method of the present invention is simpler than that of the known methods, since it is possible, in addition to the temperature, to also reduce the reaction time, another essential parameter for an industrial process.

### EXAMPLE No. 2. Synthesis of arabinofuranosyl-uracil (ARA-U)

210 grams pf cyclouridine were reacted with 100 ml NaOH 32% diluted in 1700 ml of water, for 18-20 hours at room temperature (see the diagram below).

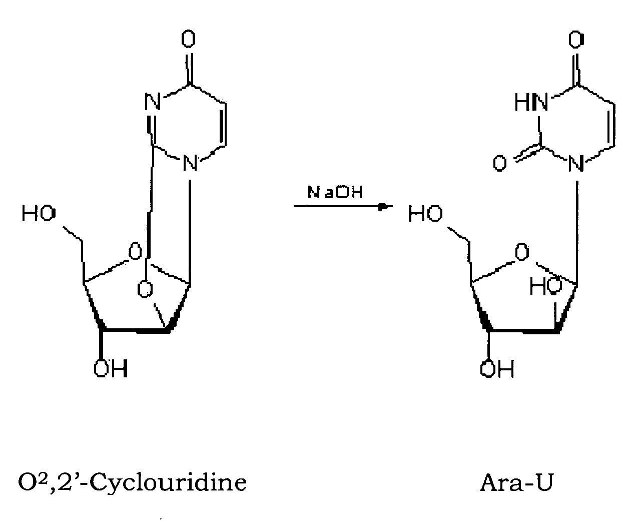

At the end of the reaction the pH of the mixture was adjusted to about 6.5 with HCl 37%, and the mixture was carbon filtered and concentrated under vacuum to a quarter of the initial volume.

The concentrated mixture thus obtained was then cooled at 4°C overnight.

The crystals contained in the cooled mixture were then filtered, washed and dried thus obtaining 146 g of Ara-U (244.202) with a purity measured by HPLC of 100%

The mother liquors obtained from the processing described above were then concentrated, diluted with ethanol, filtered and the crystalline solid recovered was dried thus allowing the recovery of 40 g of ARA-U of second extraction with 99.9% purity, giving an overall w/w yield of 88% (t.s. 82%).

## Claims

1. Method for the synthesis of possibly substituted cyclic derivatives of uridine, of formula (II) 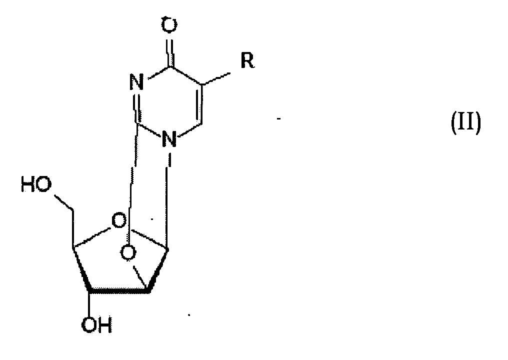 wherein R is chosen in the group that comprises: hydrogen, halogen and C₁₋₄ alkyl, the method comprising the step of reacting a possibly substituted uridine of formula (III) 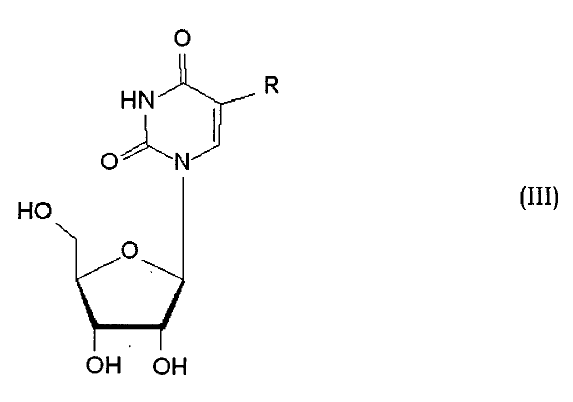 wherein R has the same meanings as in formula (II), and glycerol carbonate in the presence of an alkaline carbonate at a temperature of 70°C-130°C.

2. Method according to claim 1 wherein R is chosen in the group that comprises: hydrogen, fluorine, ethyl and methyl.

3. Method according to claim 1 or 2 wherein said possibly substituted uridine of formula (III) and the glycerol carbonate are made to react in a w/v ratio that is variable between 1:10 and 1:2.

4. Method according to any one of the previous claims wherein said possibly substituted uridine of formula (III) and the glycerol carbonate are made to react in a w/v ratio that is variable between 1:5 and 2:3.

5. Method according to any one of the previous claims wherein said possibly substituted uridine of formula (III) and the glycerol carbonate are made to react in a w/v ratio of 1:3.

6. Method according to any one of the previous claims wherein the reaction time is comprised between 0.5 and 4 hours.

7. Method according to any one of the previous claims wherein the reaction between possibly substituted uridine and glycerol carbonate is carried out at a temperature comprised between 90 and 110°C for 1-3 hours.

8. Method according to any one of the previous claims wherein the reaction between possibly substituted uridine and glycerol carbonate is carried out at a temperature of 100°C for 2 hours.

9. Method according to any one of the previous claims comprising a further final separation step of said possibly substituted cyclic derivative of uridine from the final reaction mixture, which includes the steps of: concentration of said mixture; cooling of said mixture; and dilution of said mixture in an alcohol, preferably methanol.

10. Method according to claim 9 wherein said concentration step is carried out under vacuum at a temperature comprised between 50 and 70°C.

11. Method according to claim 9 or 10 wherein said concentration step is carried out under vacuum at a temperature of 60°C.

12. Method of synthesis of derivatives of arabinofuranosyl-uracil of formula (IV) 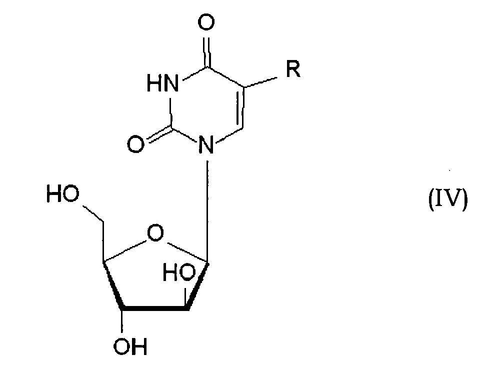 wherein R is chosen in the group that comprises: hydrogen, halogen, and C₁₋₄ alkyl, the method comprising the steps of:
a) Converting a possibly substituted uridine of formula (III) 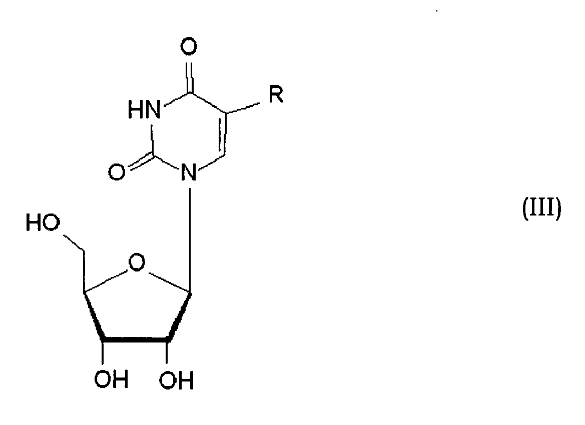 wherein R has the same meanings as in claim 1, to a possibly substituted cyclic derivative of uridine of formula (II); 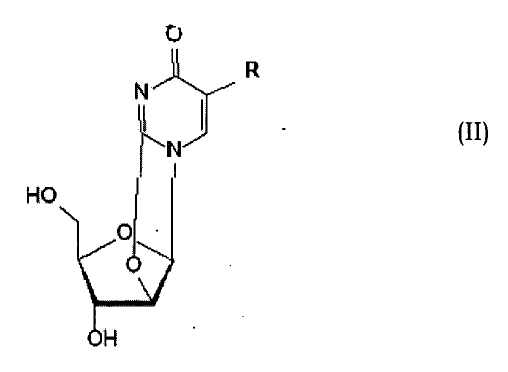
b) Reacting said possibly substituted cyclic derivative of uridine of formula (II) with an alkaline hydroxide in aqueous solution;
**characterised in that** said reaction of step a) is carried out by reacting said possibly substituted uridine of formula (III) wherein R has the same meanings as in formula (II), and glycerol carbonate in the presence of an alkaline carbonate at a temperature of 70-130°C.
